(19) Europäisches Patentamt
European Patent Office
Office européen des brevets

(11) **EP 3 911 180 B1**

(12) **EUROPEAN PATENT SPECIFICATION**

(45) Date of publication and mention of the grant of the patent:
**21.05.2025 Bulletin 2025/21**

(21) Application number: **20700507.5**

(22) Date of filing: **16.01.2020**

(51) International Patent Classification (IPC):
*A23L 33/195* (2016.01)  *A23C 9/12* (2006.01)
*C12N 9/38* (2006.01)

(52) Cooperative Patent Classification (CPC):
**A23L 33/195; A23C 9/1206; C12N 9/2471; C12Y 302/01023; C12Y 302/01108**

(86) International application number:
**PCT/EP2020/050966**

(87) International publication number:
**WO 2020/148362 (23.07.2020 Gazette 2020/30)**

(54) **PAENIBACILLUS WYNNII BETA-GALACTOSIDASE FOR THE PRODUCTION OF LACTOSE-DEPLETED AND GOS-ENRICHTED DAIRY PRODUCTS**

**PAENIBACILLUS WYNNII BETA-GALAKTOSIDASE ZUR HERSTELLUNG VON LAKTOSE-REDUZIERTEN UND GOS-ANGEREICHERTEN MILCHPRODUKTEN**

**BÊTA-GALACTOSIDASE DE PAENIBACILLUS WYNII POUR LA PRODUCTION DE PRODUITS LAITIERS APPAUVRIS EN LACTOSE ET ENRICHIS EN GOS**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.01.2019 EP 19152027**

(43) Date of publication of application:
**24.11.2021 Bulletin 2021/47**

(73) Proprietor: **Universität Hohenheim
70599 Stuttgart (DE)**

(72) Inventors:
• **FISCHER, Lutz
73274 Notzingen (DE)**
• **LUTZ-WAHL, Sabine
73230 Kirchheim unter Teck (DE)**

(74) Representative: **Müller-Boré & Partner
Patentanwälte PartG mbB
Friedenheimer Brücke 21
80639 München (DE)**

(56) References cited:
**EP-A1- 2 530 148     US-A1- 2017 164 631**

• ANONYMOUS: "A0A098MAF9_9BACL", 5 December 2018 (2018-12-05), INTERNET, pages 1 - 2, XP055593754, Retrieved from the Internet <URL:https://www.uniprot.org/uniprot/A0A098MAF9.txt?version=20> [retrieved on 20190604]

Remarks:
The complete document including Reference Table(s) and the Sequence Listing(s) can be downloaded from the EPO website

**Description**

The present invention relates to uses of an enzyme having beta-galactosidase activity and being derived from *Paenibacillus wynnii* in the production of lactose-depleted or lactose-free dairy and galacto-oligosaccharide-enriched dairy products. The present invention further relates to respective methods for the production of lactose-depleted or lactose-free and galacto-oligosaccharide-enriched dairy products.

For the enzymatic hydrolysis of milk and whey products, numerous processes with soluble and immobilized enzyme preparations have been described. If a soluble enzyme preparation is used, lactose is usually hydrolysed batchwise after pasteurization. Hydrolysis overnight at low temperatures is preferred, as this is more practicable than hydrolysis for e. g. 4 hours at 37°C, where the milk must be processed immediately after hydrolysis.

In the production of UHT (ultra-high temperature processed) milk, enzymes may be added aseptically after heat treatment. However, the requirements for enzyme purity are higher and the enzyme is present in active form in the end product.

Since the enzyme costs are critical for these processes, various immobilization methods with beta-galactosidases have been investigated. In Italy, lactose hydrolysis in milk has long been described with immobilized beta-galactosidase.

In addition to enzymatic reduction of the lactose concentration in milk and whey products, there is also the possibility of selective chromatographic separation of lactose. The world market leader in lactose-free dairy products has been using a combined process, using both enzymatic hydrolysis and selective chromatographic separation of lactose from milk to produce lactose-free milk.

In earlier studies, a new industry-relevant galactosidase has already been found in metagenome studies. A metagenome library of 1.3 million clones was screened for new beta-galactosidases using a three-stage activity-based screening. Six metagenome beta-galactosidases showed improved lactose hydrolysis in milk compared to the commercial enzyme preparation GODO-YNL2 from *Kluyveromyces lactis.* The best candidate beta-galactosidase M1 (beta-galactosidase 3J33 as described in EP 2 530 148 A1) showed a higher affinity to the substrate lactose and a lower product inhibition to the resulting galactose than the commercial enzyme preparation.

Beta-galactosidases from *Paenibacillus thiaminolyticus* and *Paenibacillus barengoltzii* have already been described in this respect. The beta-galactosidase from *P. thiaminolyticus* had a very low affinity to the substrate lactose. The $K_{M,lactose}$ value was $206 \pm 5$ m$M$. However, the determination was carried out at pH 6 and at a temperature of 37 °C. For the beta-galactosidase PbGal2A from *P. barengoltzii,* the $K_{M,lactose}$ value of $43.27 \pm 1.84$ m$M$ was significantly higher.

Thus, although a considerable number of studies have been published investigating beta-galactosidases from different sources for lactose hydrolysis, only a small number of enzyme preparations, mainly obtained from the yeasts *K. lactis* and *K. fragilis,* are applied for industrial applications. However, the industrially used enzymes possess serious disadvantages.

The beta-galactosidases utilised in the food industry are significantly inhibited by the hydrolysis-generated product D-galactose whereas the substrate affinity to the substrate lactose is not favourable (*i.e.*, the $K_M$-value for lactose is too high (>10 m$M$)). As a result, the enzymatic hydrolysis of lactose in food requires a correspondingly large amount of enzymes (cost factor) and the time required for effective lactose hydrolysis is high (time factor). The latter entails a high risk of contamination of the process.

In addition to their hydrolytic activity, beta-Galactosidases also can have a transgalactosylation activity. High transgalactosylation activity is essential to production of galacto-oligosaccharides (GOS) which have several health benefits and are recognized as prebiotics. Thus, the production of GOS is of great interest.

In this context, US 2017/0164631 A1 describes a method of producing milk products containing GOS at low temperature using beta-galactosidase enzymes having transgalactosylation activity. Further, Uniprot database entry A0A098MAF9 shows the amino acid sequence of *Paenibacillus wynnii* beta-galactosidase.

In summary, the technical problem underlying the present invention is the provision of beta-galactosidases for use in the production of lactose-depleted and lactose-free and GOS-enriched dairy products which are characterized by low product inhibition, high substrate affinity and high enzyme activity.

The solution to the above technical problem is achieved by the embodiments characterized in the claims.

In particular, in a first aspect, the present invention relates to the use of an enzyme having beta-galactosidase and transgalactosylation activity in the production of (i) lactose-depleted or lactose-free, and (ii) galacto-oligosaccharide-enriched dairy products, wherein said enzyme is derived from *Paenibacillus wynnii,* and wherein said enzyme comprises (a) the amino acid sequence of SEQ ID NO: 1; or (b) an amino acid sequence having at least 80% sequence identity to SEQ ID NO: 1 and having beta-galactosidase and transgalactosylation activity.

As used herein, the term "enzyme having beta-galactosidase activity" relates to a glycoside hydrolase enzyme that catalyses the hydrolysis of terminal non-reducing beta-D-galactose residues in beta-D-galactosides through the breaking of a glycosidic bond.

The enzyme having beta-galactosidase and transgalactosylation enzyme activity used in the present invention is either (i) the *P. wynnii* beta-galactosidase having the amino acid sequence of SEQ ID NO: 1, the advantageous use of which in the production of lactose-depleted or lactose-free dairy products, as well as in the production of GOS-enriched dairy products, has been discovered in the present invention, or (ii), in the case of an enzyme having at least 80% sequence identity to SEQ ID NO: 1 and having beta-galactosidase and transgalactosylation activity, a respective beta-galactosidase derived therefrom.

The term "lactose-depleted dairy product" as used herein relates to a dairy product whose lactose content has been reduced with respect to its original lactose content. In specific embodiments, the lactose content has been reduced by at least 75%, at least 80%, at least 85%, at least 90%, at least 95%, at least 95.5%, at least 96%, at least 96.5%, at least 97%, at least 97.2%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, or at least 99.9%.

Furthermore, the term "lactose-free dairy product" as used herein relates to a dairy product no longer containing any lactose, or containing lactose to a maximum of 1%, 0.9%, 0.8%, 0.7%, 0.6%, 0.5%, 0.4%, 0.3%, 0.2%, or 0.1% (w/v).

The terms "galacto-oligosaccharide-enriched dairy product" or "GOS-enriched dairy product" as used herein relate to a dairy product whose GOS content has been increased with respect to its original GOS content. In specific embodiments, a relative GOS yield of at least 5%, at least 10%, at least 15%, at least 20%, at least 25%, at least 30%, or at least 35%, up to e.g. 37.8%, can be achieved. Further, in specific embodiments, the GOS content in GOS-enriched dairy products according to the present invention is increased by a factor of at least 2, at least 10, at least 20, at least 50, at least 100, at least 200, at least 500, at least 1000, at least 1500, at least 2000, at least 2500, at least 3000, or more. Specific GOS contents in GOS-enriched dairy products according to the present invention are for example at least 0.1 g/L, at least 0.5 g/L, at least 1 g/L, at least 2.5 g/L, at least 5 g/L, at least 10 g/L, at least 25 g/L, at least 50 g/L, at least 75 g/L, at least 100 g/L, at least 125 g/L, at least 150 g/L, or more.

According to the present invention, the production of GOS-enriched dairy products, i.e., the increase of the GOS content in the dairy products, is achieved by way of the generation of GOS by the enzyme having beta-galactosidase activity.

In this context, the terms "galacto-oligosaccharide" and "GOS" as used herein relates to oligosaccharides that are produced from lactose by a beta-galactosidase, having 2 to 8 saccharide units, with the terminal saccharide being glucose and the remaining saccharide units being galactose. The linkages within the GOS molecule are beta-$(1\rightarrow n)$ linkages, whereas n can be 2, 3, 4 and 6. GOS display both structural and functional similarities with human breast milk oligosaccharides and, due to these similarities, are used as prebiotic in baby food.

The term "dairy products" as used herein is not particularly limited and includes a type of food produced from or containing the milk of mammals, preferably cattle, water buffaloes, goats, sheep, camels, and humans. In preferred embodiments, the dairy product is milk, preferably bovine milk.

The term "enzyme comprising an amino acid sequence having at least 80% sequence identity to SEQ ID NO: 1 and having beta-galactosidase and transgalactosylation activity" relates to polypeptides that can comprise any number of amino acid substitutions, additions, or deletions with respect to the amino acid sequence of SEQ ID NO: 1, provided that the resulting polypeptides fulfil the requirement of having at least 80% sequence identity to SEQ ID NO: 1 and retain biological activity of a beta-galactosidase and transgalactosylase. In this context, the term "retains the biological activity of a beta-galactosidase" as used herein relates to polypeptides that have at least 5%, at least 10%, at least 25%, at least 50%, preferably at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 97.5%, at least 98%, at least 98.5%, at least 99%, at least 99.5%, 100%, or more than 100% of the activity of the *P. wynnii* beta-galactosidase having the amino acid sequence of SEQ ID NO: 1, as determined in a standard beta-galactosidase activity assay known in the art.

While the number of amino acid substitutions, additions, or deletions is generally only limited by the above proviso concerning the sequence identity and biological activity of the resulting polypeptide, it is preferable that the resulting polypeptide has at least 85%, at least 90%, at least 95%, at least 97%, at least 98%, at least 98.5%, at least 99%, at least 99.25%, at least 99.5%, at least 99.6%, at least 99.7%, at least 99.75%, at least 99.8%, at least 99.85%, at least 99.9%, or at least 99.95% sequence identity to the amino acid sequence of SEQ ID NO: 1.

Means for determining the sequence identity of an amino acid sequence to a reference sequence are known in the art.

In a second aspect, the present invention relates to a method for the production of a (i) lactose-depleted or lactose-free, and (ii) galacto-oligosaccharide-enriched dairy product, comprising the step of incubating a dairy product with an enzyme having beta-galactosidase and transgalactosylation activity as defined for the uses of the present invention under conditions and for a duration of time suitable to hydrolyse lactose present in said dairy product and to generate galacto-oligosaccharides in said dairy product.

In this second aspect, all relevant definitions and limitations indicated above for the uses of the present invention apply in an analogous manner. In particular, the dairy products and the enzyme having beta-galactosidase and transgalactosylation activity are as defined above.

Means for reducing the lactose content in dairy products by way of using an enzyme having beta-galactosidase activity, as well as respective reaction conditions and incubation durations are not particularly limited and are known in the art. In specific embodiments, the methods of the present invention are performed at a temperature of 4 to 8 °C and for a duration of 24 to 72 hours.

Similarly, means for increasing the GOS content, i.e., means for generating GOS in dairy products by way of using an enzyme having beta-galactosidase and transgalactosylation activity, as well as respective reaction conditions and incubation durations are not particularly limited and are known in the art. In specific embodiments, the methods of the present invention are performed at a temperature of 4 to 8 °C and for a duration of 21 to 88 hours.

In preferred embodiments, the lactose content in the lactose-depleted or lactose-free dairy product is at most 1%, 0.9%, 0.8%, 0.7%, 0.6%, 0.5%, 0.4%, 0.3%, 0.2%, or 0.1% (w/v).

In the present invention, a beta-galactosidase derived from *P. wynnii* was produced recombinantly and characterized in detail. Surprisingly and advantageously, this beta-galactosidase is characterized by (i) a high affinity to its substrate lactose, (ii) a lack of product inhibition by galactose, (iii) a high enzyme activity, and (iv) an activity of generating GOS.

Specifically, by cultivating a recombinant strain, an active beta-galactosidase could be provided. The beta-galactosidase showed a high affinity to the substrate lactose, with a $K_{M,lactose}$-value of 0.63 $\pm$ 0.045 m$M$. The influence of galactose was investigated in Novo buffer (*i.e.*, a buffer which has a similar ion concentration to milk) at 8°C. No inhibition of beta-galactosidase up to a concentration of 200 m$M$ could be detected. During the hydrolysis of lactose in milk with an enzyme activity of 2.7 nkat$_{lactose, 8 °C}$/mL$_{milk}$, 98.1 % of the lactose had been hydrolysed after 47 h (0.86 g/L). Already after 72 h no more lactose could be detected. During hydrolysis using the industrial enzyme Opti-Lactase (with the same enzyme activity), the lactose content was 10 times higher (9.8 g/L) after 47 h. After 149 h a lactose content of 0.56 g/L could still be detected. During hydrolysis, the synthesis of galacto-oligosaccharides (GOS) was qualitatively investigated. While Opti-Lactase produced hardly any GOS and only low-molecular saccharides, beta-galactosidase from *P. wynnii* synthesized higher-molecular GOS.

For the hydrolysis of lactose in milk to be effective, the affinity of the enzyme to the substrate lactose must be high, *i.e.*, the $K_M$ value must be low. The beta-galactosidase from *P. wynnii* used in the present invention possesses a lower $K_M$ value than the beta-galactosidases known in the art and used as technical enzyme preparations (cf. Table 1). In this context, the beta-galactosidase designated as "Metagenome M1" in the following table, as well as elsewhere herein, denotes metagenome beta-galactosidase 3J33 as described in EP 2 530 148 A1.

Table 1: Overview of $K_{M,lactose}$-values from different beta-galactosidases

| Source of beta-galactosidase | $K_{M,lactose}$ | Buffer system | Temperature [°C] |
|---|---|---|---|
| *P. wynnii* | 0.63 | milk + Novo buffer | 8 |
| | 0.34-0.52 | Novo-buffer | |
| *Aspergillus oryzae* | 51.5-52.1 | Acetate buffer | 35 |
| *Bacillus circulans* | 16 | phosphate buffer | 40 |
| *K. lactis* | 30.8 | milk + water | 8 |
| | 19.8 | phosphate buffer | 37 |
| | 33.5 | milk + Novo buffer | 8 |
| Metagenome M1 | 2.10 | milk + Novo buffer | 8 |
| | 1.44 | Novo buffer | |
| *P. barengoltzii* | 43.27 | MOPS buffer | 45 |
| *P. thiaminolyticus* | 206 | phosphate buffer | 37 |

In addition the beta-galactosidase from *P. wynnii* used in the present invention is not inhibited by the hydrolysis-generated product galactose which leads to a more effective hydrolysis of lactose (cf. Table 2)

Table 2: Influence of galactose on the enzyme activity of different beta-galactosidases

| Source of beta-galactosidase | Influence of galactose (200 m$M$) | Temperature [°C] | Substrate |
|---|---|---|---|
| *P. wynnii* | 100 % EA | 8 | Lactose |

(continued)

| Source of beta-galactosidase | Influence of galactose (200 m*M*) | Temperature [°C] | Substrate |
|---|---|---|---|
| *K. lactis* | 32.5 % EA | 8 | Lactose |
| | 75 % EA | 37 | oNPGal |
| Metagenome M1 | 100 % EA | 37 | oNPGal |
| *P. barengoltzii* | 43.27 % EA | 45 | oNPGal |

During lactose hydrolysis in milk (scale of ~30 mL), which was carried out at 5 to 8 °C using beta-galactosidase from *P. wynnii* according to the present invention and Opti-Lactase (*K. lactis*) over a period of 149 h, lactose was degraded significantly faster using beta-galactosidase from *P. wynnii.* After 47 h, 98.1 % had been hydrolysed. This corresponded to a concentration of 0.86 g/L. After 72 h no more lactose could be detected by HPLC (detection minimum 0.45 g/L). In comparison to this, using Opti-Lactase, after 47 h a ten times higher lactose concentration was present (9.8 g/L). After 149 h a lactose concentration of 0. 56 g/L could be determined (cf. the Examples, below).

The present invention discloses the following amino acid sequences:

*P. wynnii* beta-galactosidase (SEQ ID NO: 1)

```
MRKKLVYSPP  TNGYPEWNNN  PECFQINRMD  AHATWIPFNT  TEDALLGDPQ  SSPNYLSLNG

MWKFAYAETP  DQRIRNFFEK  NYDCSSWAEL  NVPSHWQMHG  YDYPQYTNVR  YPWSEREPEL

KPPFAPTQYN  PVGSYVRTFS  VPEDWSGKPV  FISFQGVESA  FYVWLNGELV  GYSEDTFTPA

EFDLTPYLIP  GDNKLAVEVY  RWCDASWLED  QDFWRLSGIF  RDVYLYTTPE  AHIYDFFVRT

ELDEQYRHAE  LQLDVKLMDY  FERTAEAVIV  HAQLYDHDQN  AIFDQPLSQT  VYFNSASTQT

LQFSSSIIDP  LKWSAEHPNL  YTLVLSLHHV  DGELMEAVSC  RVGFRTFELK  DGLMKINGKR

IVFKGVNRHE  FSCDTGRSID  VDDMVRDILL  MKAHNINAVR  TSHYPNQTIW  YDLCDQYGLY

VIDETNLETH  GSWSYGQTDL  GGNTVPGSRP  EWRANVLDRC  NSMLQRDKNH  PSIVIWSLGN

ESFGGDNFVA  MHDFLKKEDP  SRLVHYEGLF  HYRESDVASD  IESTMYISPA  DVEQYALNDP

KKPYILCEYS  HAMGNSCGGL  HLYWEVFEKY  DILQGAFIWD  WIDQAIRLKQ  ADGSMHMAYG

GDFGESPHDG  NFCGNGLIFA  DRSVSPKLYE  VKKCYQNVKF  EAVDLERGIY  RVTNQNLFTD

LAEYALAWEV  SCNGNPVLKG  TVDLAVPAGE  SAEISVPVVD  EPNLQSEGEH  VLTFSLQLKK

STLWADAGHE  VAWEQFLLPT  PQFMAGQDQD  SVLTSDRGVI  VEEQAGRLTV  QAADVSLQFS

TSSGYLISMQ  NKGKELLLEP  VRPNFWRAVT  DNDLGNKHHE  RCAVWNTAGA  GCTLASFESH

KNVDGVTVRA  KYTVPTVPIS  SLILEYRIQE  NGSIEVFEEL  SPGMGLPEIP  EIGLMFIVED

RLDTVSWYGR  GPHENYWDRK  TGARLGYFSG  SVQDQFVPYI  RPQECGNKTD  VRFASITGGI

NGSGFRVDGD  PVLELNALPW  TPAELEANDH  IYKLPASNKT  VVRVNYKQMG  VGGDNSWGAT
```

*P. wynnii* beta-galactosidase coding sequence (SEQ ID NO: 2)

```
atgcgtaaaa aacttgtcta ttcaccccct accaacgggt acccggaatg gaataataat
cccgaatgtt ttcagatcaa ccgaatggat gcccatgcta catggatacc ttttaataca
acagaggatg ccttgcttgg agatccacaa tcctcgccca actatttgtc tttgaatggg
atgtggaaat tcgcctatgc agagactccg gatcaacgca tacgtaactt ttttgaaaaa
aactatgatt gcagttcttg ggctgaactg aacgttccct ctcattggca gatgcatgga
tatgactacc ctcagtatac gaatgtacgg tatccctgga gtgaacgtga gccggagctc
aaaccgcctt ttgctcccac ccaatataat ccggttgggt cttatgtacg aaccttctct
gtaccggagg actggagcgg gaagcctgtt ttcatcagct ttcaaggtgt ggagtctgcc
ttctatgttt ggctcaatgg agagttggtc ggatacagcg aggatacctt cacccctgcg
gaatttgact taactcctta tctaatcccg ggagacaata agcttgccgt tgaagtgtat
cgttggtgtg acgccagttg gctggaggat caggatttct ggagacttag cggcatattt
agagatgtct atctatatac aacaccggaa gcccatattt atgatttctt tgttcgaaca
gaactggatg agcagtaccg gcatgctgaa ttgcagctgg atgtgaagct tatggattat
tttgaaagaa ccgctgaggc agttattgtg catgcgcagc tttatgatca cgatcagaac
gctatctttg atcagccgct ttcacagaca gtctacttca atagcgcttc tacccagacg
ctccaattct cttcttctat tattgacccg ttaaaatgga gtgcggaaca tccgaatcta
tatacgttag tgttgtcttt acatcatgtg gatggagagc tcatggaagc cgttagctgc
cgtgttggct ttcgtacatt cgagctaaag gacggtttaa tgaagattaa tggaaaacgg
atcgtcttta agggcgtgaa ccgccatgaa ttctcatgcg ataccggcag atcaattgat
gtcgatgata tggtacggga cattctactg atgaaagcac acaacatcaa tgccgttcgg
acttcgcatt atccgaatca gaccatatgg tatgaccttt gtgaccaata tgggctgtat
gtaattgatg agaccaatct ggagacacat ggttcgtgga gctatggaca gacagactta
ggcggaaata cggtacccgg cagtagacct gaatggcgcg cgaatgtact ggatcggtgt
aattcaatgc tccaaaggga caaaaaccac ccttccatcg tcatctggtc actcggtaac
gagtcctttg gcggtgataa ctttgtggct atgcatgatt ttcttaagaa agaagacccc
tcccggcttg tccattatga aggcctcttt cactatcgcg agagcgatgt tgcctcggac
attgagagca ccatgtacat tagtccggcc gatgtggaac agtatgcgct taatgatcca
aagaagcctt atattctatg cgagtatagc catgcaatgg caactcctg cggggggattg
catctatact gggaagtttt tgagaaatat gatattttac agggtgcctt tatctgggac
tggattgatc aagcgattcg acttaagcaa gccgacggat ccatgcatat ggcctacggc
ggagacttcg gggaatcccc tcatgacggc aacttctgcg aaatggctt gattttcgct
gaccgttcag tatctcctaa gctgtatgag gtcaagaaat gctaccagaa tgttaaattt
gaggctgtcg atttagagcg aggcatttat cgggtaacca atcagaatct atttacggat
ttggcggagt acgcactggc ttgggaagta agctgtaacg gcaatcctgt gcttaagggt
actgttgatc tggctgtccc tgcaggagaa tcggcagaaa tttcggttcc cgtggtcgat
```

```
gaacctaacc tgcaatccga gggcgagcat gtcctcacgt ttagcctgca attgaagaaa
tccacccttt gggcagacgc aggccatgaa gtggcatggg aacaattcct gctgcctaca
cctcagttta tggcgggtca ggatcaagac tctgttttga catcagatcg tggtgtgatt
gtggaggaac aagcagggcg tttaactgtt caggcggcgg atgtctccct tcaattcagt
acttcaagcg gttatctgat ctctatgcaa aataaaggca aggaactgct gctcgaacct
gttcgtccga atttctggcg tgcagttacg gataacgatc tagggaacaa acaccatgag
cgttgcgcgg tttggaatac agcgggagct ggctgtacac tagcttcctt tgaatcgcat
aagaatgtag atggcgtaac tgttcgtgct aagtacaccg tacccaccgt acccatttct
tcactgatat tggaatatag aatccaagaa aatggctcca ttgaagtctt tgaagagcta
tcaccgggca tgggcttgcc ggagattcct gaaattggcc tcatgtttat cgttgaagat
cgactcgaca ccgtttcatg gtatggccgc ggaccgcatg agaattactg ggaccggaag
acaggcgcca gacttggata tttttcggga agtgtccagg atcagttcgt tccgtatata
aggcctcaag aatgcggaaa caaaacggat gtgcgttttg ccagtatcac tgggggtata
aacggctcag gattccgtgt ggatggcgat cctgtactgg aactaaatgc cttgccttgg
acacccgctg agttggaagc gaatgatcac atatataagc tgccagccag taataaaacc
gttgtgcgtg tgaactacaa gcaaatgggt gtaggtggag acaacagctg gggcgcgacg
acacatcctg aattcacttt gccggcggat caaacttatg ggtttcggtt tacgattcgg
atggtgtaa
```

The figures show:

Figure 1:
Determination of the effect on the enzyme activity of the beta-galactosidase from *P. wynnii* and Opti-Lactase using different galactose concentrations (125 m*M* and 200 m*M*) as well as different lactose concentrations (125 m*M* and 50 m*M*). The enzyme activity was measured with Novo-buffer (pH 6.7) and lactose as substrate at 8 °C.

Figure 2:
Hydrolysis of lactose in milk (31.5 mL scale) with the *P. wynnii* beta-galactosidase and the commercial beta-galactosidase Opti-Lactase. Δ - no lactose content could be determined.

Figure 3:
TLC analysis of the hydrolysis products of milk by the beta-galactosidase from *P. wynnii* (A) and by the commercial Opti-Lactase (B).

Figure 4:
Determination of the influence of galactose on the enzyme activity of M1 (100 %: 0.0125 mkat$_{Lactose,8°C}$ L$^{-1}$; Novo buffer, pH 6.75).

Figure 5:
Influence of glucose and galactose on the *P. wynnii* beta-galactosidase activity (*o*NPGal-Assay, 37 °C, 100 %: 2856 nkat$_{oNPGa}$ mL$^{-1}$).

Figure 6:
Hydrolysis of lactose in milk using the beta-galactosidase from *P. wynnii* beta-galactosidase and M1 (detection limit: 0.40 g L$^{-1}$).

Figure 7:
Thin-layer chromatography of the samples of hydrolysis of lactose in milk for qualitative evaluation (2.7 nkat$_{oNPGal,8°C}$

$mL^{-1}_{milk}$) (1: Mix of Lactose, Glucose and Galactose; 2: Glucose standard; 3: Galactose standard; 4: Mix of Lactose, Glucose and Galactose).

The present invention will be further illustrated by the following example without being limited thereto.

Examples

Example 1:

Cloning of *Paenibacillus wynnii* beta-galactosidase

The construction of the expression vector for the beta-galactosidase from *Paenibacillus wynnii* was based on the genomic DNA sequence of the beta-galactosidase from *Paenibacillus wynnii* DSM18334 (European Molecular Biology Laboratory: EMBL coding: KGE19535.1; SEQ ID NO: 2) available at the EMBL, European Bioinformatics Institute. One nucleotide was exchanged for cloning, but this did not result in an amino acid exchange. In general, standard molecular biology methods according to Sambrook and Russell (Sambrook, J.; Russell, D. W. Molecular Cloning: A Laboratory Manual; Cold Spring Harbour Laboratory Press: New York, NY, 2001; Vol. 1) were used. Cloning in pET-20b (+) was followed by transformation in *E. coli* XL-1 blue cells to introduce a C-terminal His6-tag in frame, resulting in the construct pET-20b-P.w.-beta-gal. For the expression of the *P. wynnii* beta-galactosidase, the construct was transformed in *E. coli* BL21(DE3) cells.

Example 2:

Cultivation of *E. coli* BL21(DE3) pET20b-*Paenibacillus wynnii*-beta-galactosidase

*E. coli* BL21(DE3)-pET20b-P.w.-beta-gal was cultivated in $2YT_{Amp}$ medium (16 g $L^{-1}$ tryptone, 10 g $L^{-1}$ yeast extract, 5 g $L^{-1}$ NaCl containing 100 $\mu$g $mL^{-1}$ ampicillin concentration) with 2 % glucose (w/v) in a bioreactor with 3.5 L operating volume. Before inoculating the bioreactor different precultures were prepared. A single colony of *E. coli* BL21(DE3)-pET20b-P.w.-beta-gal was picked from a LB agar plate and transferred into a prepared test tube containing 5 mL $2YT_{Amp}$ medium with 2 % (w/v) glucose and incubated on an orbital shaker at 120 rpm for 15 h (preculture I). Preculture I was transferred after 15 h into a baffled flask containing 30 mL $2YT_{Amp}$ medium and 2 % (w/v) glucose (preculture II). Preculture II was incubated on an orbital shaker at 90 rpm for 15 h before it was added to a baffled flask containing $2YT_{Amp}$ medium with 2 % (w/v) glucose (preculture III). After incubation for 15 h on an orbital shaker (80 rpm) preculture III was added sterilely to 3.5 L $2YT_{Amp}$ medium with 2 % (w/v) glucose that was prepared in a bioreactor. The following parameters were chosen for the cultivation: 300 rpm, air gassing $vvm$ = 2, and pH 7. The pH was adjusted using 2 $M$ NaOH and 2 $M$ $H_3PO_4$. The cells were cultivated at 37 °C up to an $OD_{600}$ of 5. For the expression of the recombinant protein the cells were cooled down to 30 °C and induced with 0.5 m$M$ isopropyl-beta-D-1-thiogalactopyranoside (IPTG). After further 9 h cultivation at 30 °C the cells were harvested using a continuous working centrifuge (CEPA Rapid centrifuge Type GLE, flow rate 350 mL $min^{-1}$, 36,000 g, 4 °C). The harvested cells were washed twice with saline and centrifuged for 10 min at 6,000 g and 4 °C.

100 m$M$ potassium phosphate buffer with 5 m$M$ $MgCl_2$ (pH 6.75) was added to the cells to prepare a 25 % (w/v) cell suspension. The cells were disrupted on ice using a sonificator (cycle 0.5, amplitude 95 %). Cell disruption was carried out in ten cycles of one-minute pulsed intervals followed by one-minute breaks. After disruption the cell suspension was centrifuged for 20 min at 8000 *g* and 4 °C. The cell pellet was discarded and the enzyme in the supernatant was used for further experiments.

Example 3:

Purification of the recombinant *P. wynnii* beta-galactosidase

The recombinant *P. wynnii* beta-galactosidase with a hexa histidine-tag was purified by nickel affinity chromatography (Biofox 40 IDALow XK16, 5 mL, Knauer, Berlin). The disrupted cells were centrifuged for 20 min at 4 °C and 8,000 g. The supernatant was centrifuged again for 20 min at 4 °C and 20000 g. Afterwards, the supernatant was filtrated (0.45 $\mu$m) and 5 mL of the crude extract were injected into an ÄKTA FPLC system (GE Healthcare, Freiburg). The column was previously equilibrated with 2 CV binding buffer. The conditions for the purification of *P. wynnii* beta-galactosidase are listed in Table 3. The eluted proteins were detected by an UV detector at $\lambda$ = 280 nm and fractionated in 2.5 mL fractions. The fractions in which an UV signal was detected were pooled. Size exclusion chromatography columns (PD10 columns, GE Healthcare, Freiburg) were used to change the buffer to 100 m$M$ potassium phosphate buffer (pH 6.75 containing 5 m$M$

magnesium chloride).

Table 3: Conditions for the purification of *P. wynnii* beta-galactosidase by nickel affinity chromatography

| | |
|---|---|
| Flow rate - binding | 1 mL/min |
| Flow rate - elution | 2 mL/min |
| Elution gradient | 2 CV 10 m*M* imidazole |
| | 4 CV linear 10 - 250 m*M* imidazole |
| Washing | 2 CV elution buffer |

Binding buffer: 100 m*M* potassium phosphate buffer containing 150 m*M* NaCl Elution buffer: 100 m*M* potassium phosphate buffer containing 150 m*M* NaCl and 250 mM imidazole

Example 4:

Determination of enzyme activity

The beta-galactosidase activity was determined using either the synthetic substrate *o*-nitrophenyl-beta-D-galactopyranoside (*o*NPGal) or the natural substrate of the enzyme lactose.

*Beta-galactosidase activity with o-nitrophenyl-beta-D-galactopyranoside (oNPGal) as substrate.*

The enzyme activity of beta-galactosidase was determined in 1.2 mL scale using *o*NPGal as substrate at 37 °C. For the assays a final concentration of 25 m*M o*NPGal dissolved in 100 m*M* potassium phosphate buffer (pH 6.75 containing 5 m*M* magnesium chloride) was used. Substrate and enzyme solution were preheated separately. The reaction was performed in a temperature-controlled cuvette using a spectrophotometer by adding enzyme solution to oNPGal solution. The increase of absorbance at 405 nm as result of *o*-nitrophenol release was measured for 2 min. The activity was calculated from the slope of the straight line.

One nanokatal is defined as the amount of enzyme that catalyses the release of 1 nmol of o-nitrophenol from oNPGal per second. The amount of the released *o*-nitrophenol was determined using a calibration curve (range: 0.0167-1.67 m*M o*-nitrophenol).

*Beta-galactosidase activity with lactose as substrate.*

The beta-galactosidase activity was determined using lactose as a substrate. Beta-galactosidase hydrolyses the beta-1,4-glycosidic bond in lactose and release D-glucose and D-galactose.

For the substrate solution 400 m*M* lactose was dissolved in Novo buffer (the composition of which is depicted in Table 4). The beta-galactosidase activity was determined in a thermo shaker in 1.5 mL reaction tubes at 8°C. The enzyme mix and the lactose solution were preincubated at 8 °C for 12 min. To start the enzymatic reaction 100 $\mu$L of the enzyme mix were added to 100 $\mu$L lactose solution. The reaction was carried out for 5 min at 8 °C. The reaction was stopped by adding 300 $\mu$L 1 *M* HClO$_4$ to the enzyme-substrate-mix. The mix was kept on ice for 5 min before it was centrifuged at 13 000 rcf and 4 °C for 5 min. 160 $\mu$L of the supernatant were transferred to 75 $\mu$L 2 *M* KOH to neutralise the solution. The neutralisation step was checked using pH paper and the pH was adjusted if necessary. The solution was kept on ice for another 5 min before it was centrifuged at 13 000 rcf at 4 °C for 5 min. All assays were performed in triplicate.

Table 4: Composition of Novo buffer

| Component | Concentration [mM] |
|---|---|
| Trisodium citrate | 2.7 |
| Citric acid | 7.91 |
| $K_2HPO_4$ | 2.99 |
| $KH_2PO_4$ | 10.84 |
| KOH | 19.43 |
| $MgCl_2$ | 4.08 |
| $CaCl_2$ | 5.1 |
| $Na_2CO_3$ | 3.33 |

*Enzymatic D-glucose determination.*

The glucose concentration was determined for the calculation of the beta-galactosidase activity with lactose as a substrate. The glucose concentration was determined using an enzymatic assay. The commercial kit hexokinase/glucose-6-phosphate dehydrogenase (Megazyme, Wicklow, Ireland) was used. The D-glucose is phosphorylated to glucose-6-phosophate by the enzyme hexokinase using ATP as cosubstrate. In a coupled reaction glucose-6-phosphate is oxidized to gluconate-6-phosphate by the enzyme glucose-6-phosphate dehydrogenase while $NADP^+$ is reduced to NADPH which was quantified photometrically at 340 nm. The amount of the formed NADPH equals the amount of glucose in the solution. The determination of the glucose concentration was performed using a robotic liquid handling system (MultiPROBE II EX, Packard Bioscience, Meriden, CT, USA). The hexokinase/glucose-6-phosphate dehydrogenase suspension was diluted 1:4 with $H_2O_{dd}$ before use. TRA buffer which contained $NADP^+$ and ATP was prepared according to Table 5.

Table 5: Composition of TRA buffer for the enzymatic determination of D-glucose

| Component | Concentration [g/L] |
|---|---|
| Triethanolamine hydrochloride | 33.6 |
| ATP | 2.15 |
| $NADP^+$ | 1.3 |
| $MgSO_4 \cdot 7\ H_2O$ | 0.9 |

For the glucose determination 235 $\mu$L TRA buffer were prepared in a 96 well plate and 15 $\mu$L of the sample solution was added. After 3 min the absorption at $\lambda$ = 340 nm was measured in a plate reader (A1). Subsequently, 5 $\mu$L of the diluted hexokinase/glucose-6-phosphate dehydrogenase were added to each well. The plate was shaken in the plate reader and incubated at room temperature. After 30 min the absorption at $\lambda$ = 340 nm was measured again (A2). With the absorption difference $\Delta A$ = A2 - A1 the glucose concentration was calculated using a calibration curve (range; 0.0075-1 g/L glucose).

Example 5:

Determination of kinetic parameters

The kinetic parameters were determined using the natural substrate lactose. The reaction matrices and conditions were chosen in order to simulate conditions in milk systems. The chosen reaction matrices were Novo buffer (cf. Table 4, above) and milk diluted with Novo buffer. The reaction temperature was 8 °C and the pH value was 6.7.

*Determination of* $K_M$.

For the determination of the $K_M$ values of *P. wynnii* beta-galactosidase lactose concentrations ranged from 0.76 m*M* to 97.5 m*M*. In order to obtain different lactose concentrations in milk, milk was diluted with Novo buffer. It was used a beta-galactosidase activity of 6.11 nkat$_{Lactose,8\ °C}$/mL for the determination in milk and 11.34 nkat$_{Lactose,8\ °C}$/mL in buffer. The enzyme activities were measured under initial reaction velocity conditions of the enzyme. The reaction times for the initial velocity of the enzymes were determined in previous experiments. The kinetic parameter $K_M$ was analysed using the Enzyme Kinetics Module in SigmaPlot 12.5 (Systat Software, Inc., San Jose, USA).

The $K_M$ values determined are summarized in Table 6. In the case of the studies in Novo buffer the enzyme activity decreased again with increasing lactose concentration. For this reason, in addition to the Michaelis Menten evaluation, the evaluation also included an evaluation taking into account a substrate inhibition.

Table 6: Summary of the determined $K_M$ values using the Enzyme Kinetics Module in SigmaPlot 12.5. A: Evaluation according to Michaelis-Menten, B: Evaluation under consideration of substrate inhibition.

| | $K_{M,Lactose}$ [m*M*] | $R^2$ [-] |
|---|---|---|
| Milk (A) | 0.63 $\pm$ 0.045 | 0.95 |
| Novo buffer (A) | 0.34 $\pm$ 0.084 | 0.61 |
| Novo buffer (B) | 0.52 $\pm$ 0.089 | 0.80 |

*Influence of galactose on the enzyme activity.*

The influence of galactose was investigated with *P. wynnii* beta-galactosidase and the commercial beta-galactosidase Opti-Lactase (Optiferm GmbH, Oy-Mittelberg (D)). The determination of the enzyme activity in Novo buffer was conducted with a lactose concentration of 125 m*M* and 50 m*M* respectively. To this solutions 125 or 200 m*M* galactose were added. The determination of the enzyme activity was performed as described above.

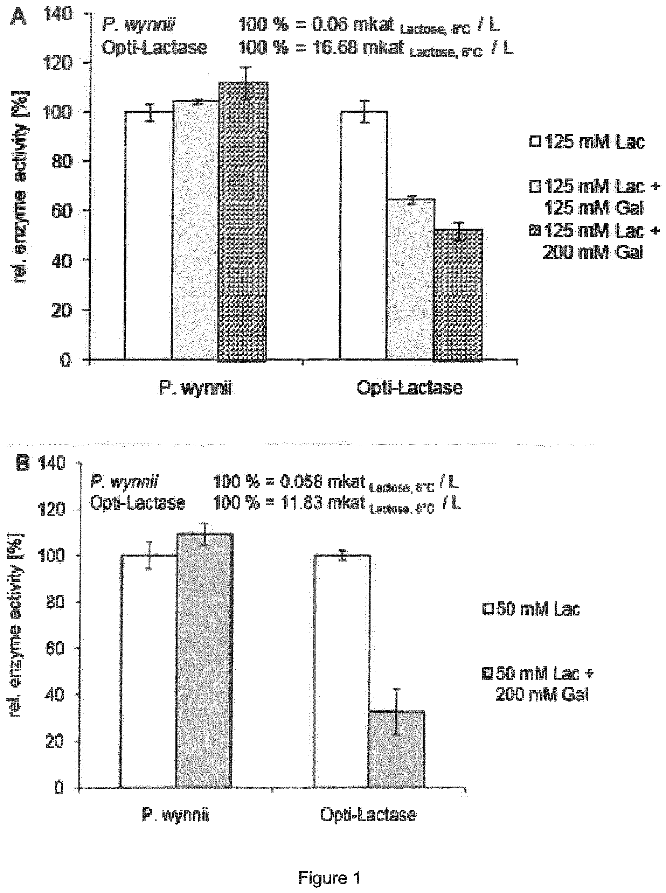

Figure 1 shows the relative enzyme activity of the two beta-galactosidases. First, 125 and 200 m*M* galactose respectively were added to 125 m*M* lactose (A). The enzyme activity of the technical enzyme preparation decreased with increasing galactose concentration. An activity of 51.8 % was determined. In contrast, galactose did not have an inhibitory effect on the activity of the beta-galactosidase from *P. wynnii*. In another experiment 200 m*M* galactose was added to 50 m*M* lactose (B). Here, the same effect as in (A) could be observed. Furthermore, the comparison of enzyme activity at 125 and 50 mM showed that with lower lactose concentrations the activity of Opti-Lactase decreased significantly. The determined enzyme activity decreased from $16.68 \pm 0.76$ mkat$_{Lactose,8\ °C}$/L to $11.84 \pm 0.23$ mkat$_{Lactose,8\ °C}$/L. The enzyme activity of the beta-galactosidase from *P. wynnii* did not change significantly ($59.9 \pm 2.0$ µkat$_{Lactose,8\ °C}$/L).

Example 6:

Comparison of lactose hydrolysis of *P. wynnii* beta-galactosidase and commercial preparations of Opti-Lactase

The hydrolysis of lactose in milk was performed both with the beta-galactosidase from *P. wynnii* and with the technical enzyme preparation Opti-Lactase (*K. lactis*). The hydrolysis was conducted in a 31.5 mL scale at approx. 5 to 8 °C. For the conversion an enzyme activity of 2.7 nkat$_{Lactose,\ 8\ °C}$/mL milk was used. Samples were taken over a period of 149 h. Therefore 200 µL samples were taken and stopped with perchloric acid as described above and the lactose content was determined by HPLC (cf. Example 7, below). GOS synthesis was investigated by HPLC and thin-layer chromatography (cf. Example 8, below).

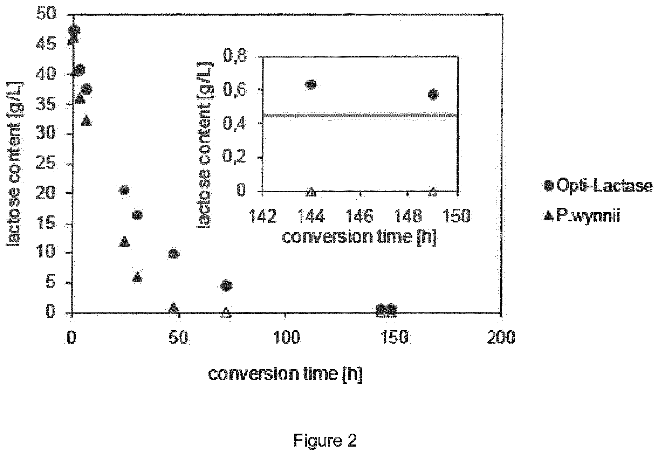

Figure 2 shows the course of the lactose hydrolysis in milk over time. The beta-galactosidase from *P. wynnii* exhibited a better performance in lactose hydrolysis from the beginning. After 24 hours, 75% of the lactose had already been hydrolysed with *P. wynnii* beta-galactosidase compared to 56% with Opti-Lactase. After 47 h 98.1 % lactose was hydrolysed which corresponds to a residual milk lactose content of 0.86 g/L with P. wynnii beta-galactosidase and after 72 h no more lactose could be detected by HPLC (detection limit 0.45 g/L). In comparison to *P. wynnii* beta-galactosidase, with Opti-Lactase lactose amounts of 9.8 g/L after 47 h, 4.6 g/L after 72 h, and 0.56 g/L even after 149 h could be determined.

Example 7:

HPLC analysis

An Agilent Series 1100 HPLC system, equipped with a Shodex HILICpakVG-50 4E column (4.6 × 250 mm, 5 µm, Shodex) and a low-temperature evaporative light scattering detector (ELSD Sedex 85LT, Sedere, Alfort ville Cedex, France) at 50 °C and 3 bar was used to determine the concentration of lactose. The column was eluted with 75 % (v/v) acetonitrile, 15% (v/v) methanol, and 10% (v/v) double-distilled water at a flow rate of 0.75 mL/min at 40 °C.

Example 8:

Thin layer chromatography

Thin layer chromatography was used in order to visualize the products (lactose, glucose, galactose and galacto-oligosaccharides (GOS)) generated from milk lactose by enzymatic conversion with the two beta-galactosidases (cf. Example 6, above). Aliquots of 1 µL of the sample prepared were spotted on TLC plates (Silica gel 60F254, Merck, Whitehouse Station, NJ), and developed in a solvent system of 1-butanol/2-propanol/water (3:12:4). The plates were developed by a colouring agent (6.5 m*M* N-1-naphthyl ethylenediamine dihydrochloride and 3 % sulfuric acid in methanol) at 100 °C until the spots became visible.

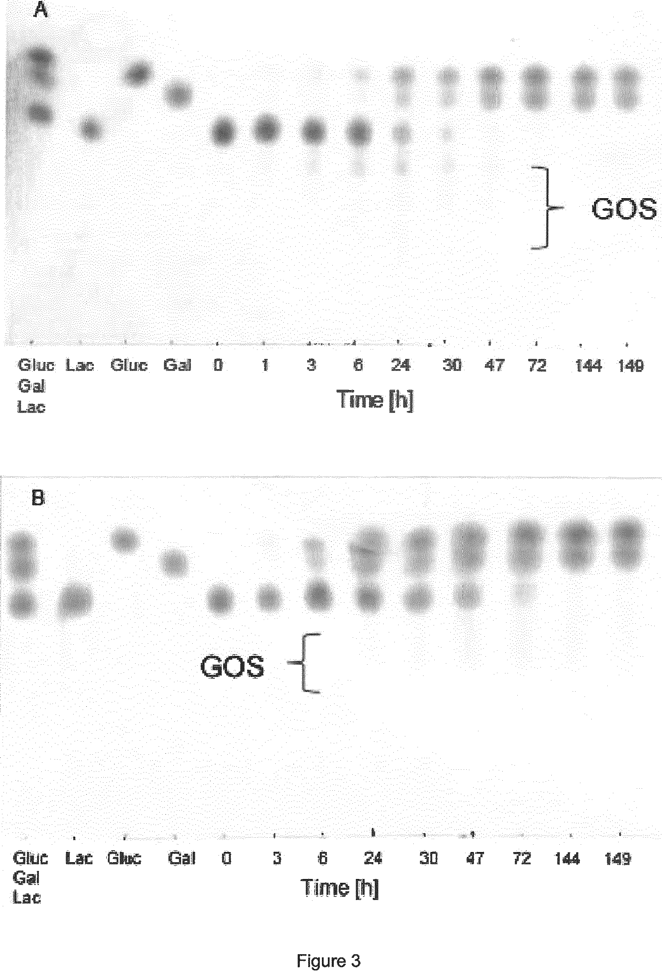

Figure 3 A shows the HPLC analysis of the samples from lactose hydrolysis by means of Opti-Lactase and Figure 3 B the analysis using beta-galactosidase from *P. wynnii.* In addition to the decrease in lactose and the increase in galactose or glucose, the formation of GOS over time can be observed. It was obvious that the GOS synthesis during the hydrolysis of lactose with Opti-Lactase occurred much later than during the hydrolysis with *P. wynnii* beta-galactosidase. While during the conversion of lactose by Opti-Lactase only a minimal GOS formation could be detected after 24 h, the conversion by *P. wynnii* beta-galactosidase showed the formation of GOS after 1 h already. The highest yield of GOS was approximately 72 h for the conversion with Opti-Lactase and 24 h with *P. wynnii* beta-galactosidase. However, the yield of Opti-Lactase was qualitatively lower than that of *P. wynnii* beta-galactosidase. In addition, different GOS were formed. While Opti-Lactase synthesized rather low molecular weight GOS, *P. wynnii* beta-galactosidase generated also higher molecular weight GOS.

Example 9:

Comparative studies of metagenome beta-galactosidase M1 and *P. wynnii* beta-galactosidase according to the present invention

The metagenome beta-galactosidase was produced and purified using nickel affinity chromatography as described above (cf. Example 3). After purification, the specific activity of M1 beta-galactosidase was 1854 nkat$_{oNPGal,37°C}$ mg$^{-1}$.

The further investigations with M1 focused mainly on a comparison of the technical applicability for the hydrolysis of lactose in milk. Therefore, the influence of galactose and glucose on the enzyme activity was investigated, as well as the hydrolysis of lactose in milk which was performed under the same conditions as the hydrolysis with *P. wynnii* beta-galactosidase of the present invention. In addition, the stability of the two enzymes was investigated.

*Influence of galactose on the enzyme activity.*

Substrate lactose

The influence of galactose was also investigated with the metagenome M1 beta-galactosidase as described before. The determination of the enzyme activity in Novo buffer was conducted with a lactose concentration of 125 m*M* and 50 m*M* respectively. To this solutions 125 or 200 m*M* galactose were added.

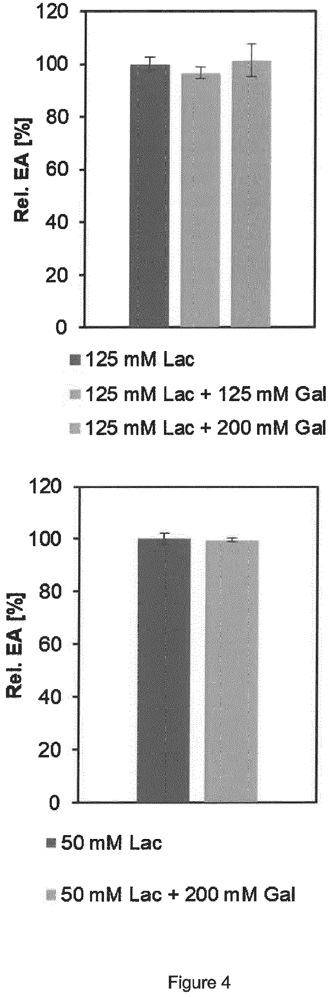

Figure 4 shows the relative enzyme activity of the beta-galactosidase M1. In both cases, the enzyme activity of the beta-galactosidase M1 did not change significantly. This means galactose did also not have an inhibitory effect on the M1 beta-galactosidase activity.

In table 7, the comparison of the results of the investigations of beta-galactosidase M1 and *P. wynnii* is depicted. With M1, no influence of galactose on activity was observed, with *P. wynnii* beta-galactosidase galactose a slightly activating effect on enzyme activity could be shown.

Table 7: Comparison of the influence of galactose on the beta- galactosidases M1 and *P. wynnii* (Lactose-Assay, 8 °C)

| | Relative enzyme activity [%] | |
| --- | --- | --- |
| | beta-galactosidase | |
| | M1 | *P. wynnii* |
| 125 m*M* lactose + 125 m*M* galactose | 97 | 108 |
| 125 m*M* lactose + 200 m*M* galactose | 101 | 115 |
| 50 m*M* lactose + 200 m*M* galactose | 100 | 110 |

Substrate *o*-nitrophenyl-beta-D-galactoside (*o*NPGal)

In addition to galactose, the effect of released glucose on the enzyme activity of the two beta-galactosidases was also of interest. Due to the experimental setup (analysis), it was not possible to measure the influence of glucose with the natural substrate lactose. Therefore, *o*NPGal was used for the determination of beta-galactosidase activity in the presence of glucose as known in the art. In addition, the influence of galactose and the influence of an equimolar concentration of both glucose and galactose was tested.

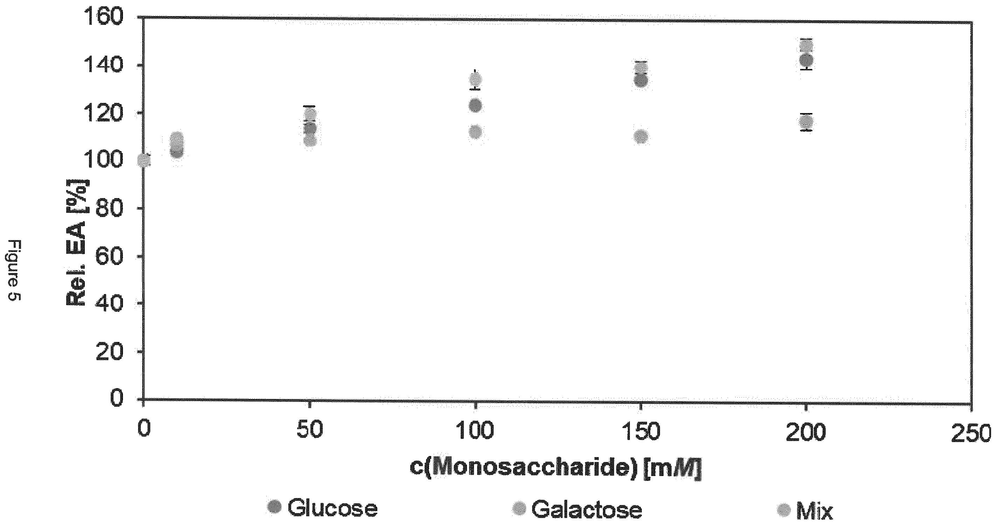

The enzyme activity of *P. wynnii* beta-galactosidase increased with increasing galactose concentration (200 m*M*: 118 % activity) as seen in Figure 5. The presence of glucose showed a much stronger activating effect on the enzyme activity of *P. wynnii* beta-galactosidase (at 200 m*M* glucose: 144% activity) compared to beta-galactosidase M1 (cf. Table 8). Only a slight improving effect on the enzyme activity (at MIX-200: 150% activity) was observed for the equimolar mixture of glucose and galactose, compared to the exclusive addition of glucose.

In summary, *P. wynnii* beta-galactosidase showed an activating effect in the presence of both glucose and galactose, while M1 only in the presence of glucose also showed an activating effect, which is less turned out. In the presence of galactose, M1 does not show inhibition but is not activated either.

Table 8: Comparison of the influence of glucose and galactose to *P. wynnii* and M1 beta-galactosidase

| | Relative enzyme activity [%] | |
| --- | --- | --- |
| | *P. wynnii* | M1 |
| 200 m*M* Glucose | 144 | 124 |
| 200 m*M* Galactose | 118 | 100 |
| *200* m*M* Mix | 150 | 124 |

*Lactose hydrolysis in milk*

The hydrolysis of lactose in milk was performed both with the beta-galactosidase M1 and the *P. wynnii* beta-galactosidase. The hydrolysis was conducted as described previously in a 31.5 mL scale at approx. 5 to 8 °C. For the conversion, an enzyme activity of 2.7 nkat$_{Lactose, 8 °C}$ mL$^{-1}$ milk was used. Samples were taken over a period of 144 h.

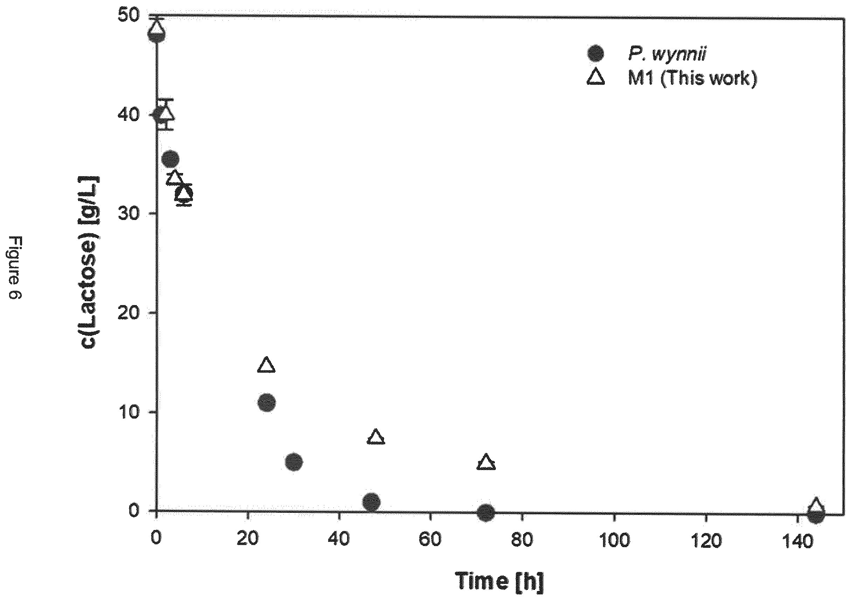

In Figure 6, the results of lactose hydrolysis with both the *P. wynnii* beta-galactosidase and M1 were presented for comparison. Both enzymes showed a similar course of lactose in the first 6 hours. However, less lactose was hydrolysed by M1 after 24 hours. After 144 h, 0.71 g L$^{-1}$ lactose was still detected, whereas with *P. wynnii* beta-galactosidase no lactose could be detected after 68 h. One reason for the better conversion of *P. wynnii* beta-galactosidase over time could be related to the higher activation of glucose and galactose released by the lactose hydrolysis. Another reason could be a lower stability of M1.

*Storage stability*

The storage stability of the two beta-galactosidases M1 and *P. wynnii* were investigated in NOVO buffer at 4°C. Already after 9 days, only a residual activity of 37% could be determined with M1, while with *P. wynnii* beta-galactosidase no loss of activity should be determined. After 18 days a residual activity of 92% could be determined with *P. wynnii* beta-galactosidase.

*Turnover number*

To determine the turnover number, the specific activity of the enzymes on lactose at 8 °C was determined. The following formula was used to determine the turnover number:

$$K_{cat} = \frac{Vmax}{[E]} \quad [s^{-1}]$$

$K_{cat}$ = turnover number [s$^{-1}$]
$V_{max}$ = maximum activity [nkat/mg]
[E] = enzyme concentration [mg/nmol]

In Table 9, the calculated turnover numbers of the two beta-galactosidases are depicted.

Table 9: Turnover numbers of the beta-galactosidases M1 and *P. wynnii* (8 °C, lactose as substrate)

| M1 | *P. wynnii* |
| --- | --- |
| 2.466 s$^{-1}$ | 5.034 s$^{-1}$ |

The turnover number of *P. wynnii* beta-galactosidase is twice as high compared to the turnover number of M1. This shows also the higher efficiency of the *P. wynnii* beta-galactosidase.

*Conclusion*

In conclusion, it could be shown that the *P. wynnii* beta-galactosidase of the present invention could be activated in the presence of glucose and galactose while the metagenome M1 beta-galactosidase was not inhibited by galactose but only activated by glucose, wherein the activation was lower than with the *P. wynnii* beta-galactosidase. The *P. wynnii* beta-galactosidase of the present invention showed a total lactose conversion in milk after 68 h while M1 had not completely hydrolysed the lactose after 144 h (98.5 % conversion) under the same conditions. It could also be shown that the *P. wynnii* beta-galactosidase is more stable than M1.

Altogether *P. wynnii* beta-galactosidase showed more favorable characteristics of the technical applicability for the hydrolysis of lactose in milk than M1.

Example 10:

Bioconversion of pharma-lactose to produce galacto-oligosaccharides with *P. wynnii*-beta-galactosidase and Opti-B

In further studies it could also be shown that the beta-galactosidase from *P. wynnii* according to the present invention is able to produce galacto-oligosaccharides (GOS). In this comparative study, the experimental Enzyme B (Opti-B) which is distributed by Optiferm GmbH, Germany, was used. This enzyme is originated from *B. circulans.* Compared to a commercially available enzyme, the GOS yield is similar and the remaining lactose content is lower. As further enzyme preparation, Saphera 2600L was used which is an industrial beta-galactosidase preparation from Novozymes A/S. The beta-galactosidase is originated from *Bifidobacterium bifidum.* The preparation is used in batch and aseptic application for the hydrolysis of lactose in dairy products.

With these and the following (cf. Example 11, infra) experiments, it could be shown that *P. wynnii* beta-galactosidase of the present invention shows both a good performance of lactose hydrolysis in milk and at the same time a good transgalactosylation potential for the formation of GOS.

Since it was shown in preliminary experiments that GOS were formed only with *P. wynnii* beta-galactosidase and Opti-B and not with Saphera 2600L, the bioconversions were carried out only with the first two. Pharma-lactose is highly purified lactose (99.93 % lactose) and was used for the bioconversions to exclude as far as possible the components of lactose influencing the performance of the beta-galactosidases.

*Bioconversion*

For the determination of the transgalactosylation activity of the *P. wynnii* beta-galactosidase, pharma-lactose (400 g L$^{-1}$) was dissolved in potassium phosphate buffer (concentrations varying from 50 to 150 m$M$) containing 5 m$M$ MgCl$_2$ (pH 6.75) at 95 °C. After cooling down, the solution (V: 40 mL) was transferred to a small reaction vessel (50 mL) which was tempered to 40 °C. To start the reaction, the beta-galactosidase (2.5 U$_{lactose,40\ °C}$ mL$^{-1}$) was added to the solution. On several time points, samples (500 $\mu$L) were taken and heated to 95 °C for 5 min to inactivate the beta-galactosidase. The samples were stored at -20 °C and used for further determination of the sugar concentrations (cf. below). For the determination of the residual activity, samples (1000 $\mu$L) were taken after 5 min, 4 h, 8 h and 24 h and the buffer was exchanged using PD10 columns to remove the sugars. Afterwards, the residual beta-galactosidase activity was measured by the *o*NPGal assay.

For a possible further application of *P. wynnii* beta-galactosidase, the production of galacto-oligosaccharides was done without buffer salts. Therefore, pharma-lactose (400 g L$^{-1}$) was dissolved in H$_2$O$_{dd}$. Also, 2.5 U$_{lactose,40\ °C}$ mL$^{-1}$ were applied. The remaining procedure was done as described above.

To compare the transgalactosylation activity of *P. wynnii* beta-galactosidase, the bioconversions were also done with the enzyme preparation Opti-B (Optiferm GmbH).

*Determination of lactose, glucose and galactose using HPLC*

Before using HPLC, 190 $\mu$L of the sample was added into a 1.5 mL reaction tube and was mixed with 10 $\mu$L fructose (200 g L$^{-1}$) as internal standard. Afterwards, 200 $\mu$L of the mixture was transferred into a HPLC vial. If necessary, the samples were diluted with H$_2$O$_{dd}$ before mixing with the internal standard.

Sugars were separated for quantification using high pressure liquid chromatography (HPLC). Therefore, two consecutive carbohydrate Ca$^{2+}$-HPLC-columns (250 x 8 mm; CS, Langerwehe, Germany) were used. Molecule separation was based on size and the different interactions of the hydroxy groups of the sugar molecules with the Ca$^{2+}$-ions of the columns.

The columns were attached to an Agilent 1100 Series HPLC system (Agilent Technologies, Santa Clara, USA) equipped with an evaporative light scattering detector (ELSD Sedex 85LT; Sedere, Olivet, France) which was operated at 50 °C and 3.5 bar for detection. The columns were tempered to 85 °C using the Croco-Cil HPLC column oven (SCO Seitz Chromatographie Produkte GmbH, Weiterstadt, Germany). The system was operated with a pressure of 80 bar. An automated sampler applied 10 $\mu$L sample on the columns. Elution was carried out with a constant flow rate of 0.5 mL min$^{-1}$ of H$_2$O$_{dd}$ for 35 min.

The parameters of the used method are listed in the following Table 10.

Table 10: Parameters of the HPLC method to measure the lactose, glucose and galactose content

| Parameter | Description |
|---|---|
| HPLC - System | Agilent 1100 Series |
| Stationary Phase | Carbohydrate $Ca^{2+}$ |
| Mobile Phase | $H_2O_{dd}$ (isocratic) |
| Flow rate | 0.5 mL $min^{-1}$ |
| Pressure | 80 bar |
| Temperature | 85 °C |
| Injection volume | 10 $\mu$L |
| Detection | ELSD Sedex 85LT |
| Total program time | 35 min |

The area of the peaks corresponded to respective analyte amounts, defined by calibration. For the preparation of the calibration curves, lactose (1.66 - 25 g $L^{-1}$), glucose (0.3 - 30 g $L^{-1}$) and galactose (0.3 - 30 g $L^{-1}$) were dissolved in $H_2O_{dd}$. The limit of detection (LOD) of lactose, glucose and galactose are 1.0 g $L^{-1}$, 0.42 g $L^{-1}$ and 0.76 g $L^{-1}$, respectively. For each sample, an individual correction factor was calculated and multiplied with the peak area of lactose, glucose and galactose, respectively. The correction factor was calculated by dividing the mean value of all fructose peaks (internal standard) of a measurement series by the peak of fructose in the corresponding sample.

*Calculation of lactose conversion and GOS yield*

After determination of the sugar concentrations using HPLC, two different parameters can be calculated.
The lactose conversion describes how much lactose was processed by the beta-galactosidase during the bioconversion and is calculated with following equation:

$$x = \left(\frac{L_0 - L_t}{L_0}\right) \cdot 100\ \% \qquad \text{(Equation 1)}$$

With:

X     = Lactose conversion [%]
$L_0$     = Lactose concentration at the beginning [g $L^{-1}$]
$L_t$     = Lactose concentration at time t [g $L^{-1}$]

The GOS yield describes the GOS concentration as a percentage of initial lactose concentration.

$$y = \left(\frac{c_{Lac,0} - \left(c_{Gal,t} + c_{Glu,t} + c_{Lac,t}\right)}{c_{Lac,0}}\right) \cdot 100\ \% \qquad \text{(Equation 2)}$$

With:

Y               = GOS yield [%]
$c_{Lac,0}$          = Lactose concentration at the beginning [g $L^{-1}$]
$C_{Lac}/_{Glu}/_{Gal,t}$     = Sugar concentration at time t [g $L^{-1}$]

Bioconversion with *P. wynnii* beta-galactosidase

The GOS production with *P. wynnii* beta-galactosidase was done under different process conditions to investigate the influence of the different parameters on GOS yield and lactose conversion. Therefore, the salt and the enzyme concentration were varied, respectively. The results of the bioconversions are shown in Table 11.

15

Table 11: Summary of the different bioconversions of pharma-lactose with *P. wynnii* beta-galactosidase (40 °C, 40 % (w/v) pharma-lactose, 24 h)

| | 1 | 2 | 3 | 4 | 5 |
|---|---|---|---|---|---|
| System | $H_2O_{dd}$ (pH 6.75) | 100 m$M$ potassium phosphate + 5 m$M$ $MgCl_2$ (pH 6.75) | 100 m$M$ potassium phosphate + 5 m$M$ $MgCl_2$ (pH 6.75) | 50 m$M$ potassium phosphate + 5 m$M$ $MgCl_2$ (pH 6.75) | 150 m$M$ potassium phosphate + 5 m$M$ $MgCl_2$ (pH 6.75) |
| Applied enzyme activity | 2.5 $U_{lactose}$ mL$^{-1}$ | 2.5 $U_{lactose}$ mL$^{-1}$ | 1.25 $U_{lactose}$ mL$^{-1}$ | 2.5 $U_{lactose}$ mL$^{-1}$ | 2.5 $U_{lactose}$ mL$^{-1}$ |
| Lactose conversion [%] | 47.0 | 65.5 | 42.7 | 58.5 | 70.1 |
| Theoretical GOS yield [%][1] | 29.0 | 36.0 | 30.0 | 27.5 | 37.8 |

The highest lactose conversion (70.1 %) and theoretical GOS yield (37.8 %) was determined when pharma-lactose was dissolved in 150 m$M$ potassium phosphate + 5 m$M$ $MgCl_2$ (pH 6.75) and 2.5 $U_{lactose}$ mL$^{-1}$ was applied. Dissolving the pharma-lactose in $H_2O_{dd}$ resulted in a lower lactose conversion as well as in a lower GOS yield, although nearly the same residual activity than in 150 m$M$ buffer was measured after 24 h. Therefore, it is assumed that potassium and phosphate ions as well as $MgCl_2$ may influence the conformation and activity of the beta-galactosidase.

Bioconversion with Opti-B

The results of the bioconversions with Opti-B are summarized in Table 12. Since the preparation Opti-B was developed to produce GOS, it was used in this study for comparison of the transgalactosylation activity. Thus, two bioconversions in buffered and unbuffered system were conducted.

Table 12: Summary of the different bioconversions of pharma-lactose with Opti-B (40 °C, 40 % (w/v) pharma-lactose, 24 h)

| | 1 | 2 |
|---|---|---|
| System | $H_2O_{dd}$ (pH 6.75) | 100 m$M$ potassium phosphate + 5 m$M$ $MgCl_2$ (pH 6.75) |
| Applied enzyme activity | 2.5 $U_{lactose}$ m L$^{-1}$ | 2.5 $U_{lactose}$ mL$^{-1}$ |
| Lactose conversion [%] | 71.4 | 61.8 |
| Theoretical GOS yield [%][1] | 38.8 | 42.7 |

When pharma-lactose was dissolved in $H_2O_{dd}$, more lactose was converted during the process while a minor GOS yield was reached. This may be due to a higher water availability in the salt-free system and thus the beta-galactosidase kinetic was shifted towards the hydrolysis reaction.

Example 11: Bioconversion in milk

In this experiment, all three enzyme preparations, the *P. wynnii* beta-galactosidase of the present invention, Opti-B and Saphera 2600L were used.

*Hydrolysis of lactose in milk*

The hydrolysis of lactose in milk was done in a small Schott bottle (V: 50 mL). Therefore, 31.5 mL milk (H-milk, "Schwarzwaldmilch", 3.8 % fat) was incubated on a magnetic stirrer at 4 - 8 °C. To prevent microbial growth, 0.1 % (w/v) sodium azide was added. To start the reaction 2.7 nkat$_{oNPGal,8°C}$ mL$^{-1}$ of the respective beta-galactosidase was added. At several time points samples (1000 $\mu$L) were taken and heated for 5 min at 95 °C to inactivate the enzyme. Afterwards, the samples were stored at -20 °C until analysis. The lactose concentration was measured after sample preparation by Carrez Clarification using HPLC (see above). For qualitative analysis, a thin-layer chromatography was done as described before.

To compare the performance of the *P. wynnii* beta-galactosidase of the present invention, this experiment was also done with the industrial enzyme preparations Opti-B (Optiferm GmbH) and Saphera 2600L (Novozymes A/S).

*Sample preparation via Carrez precipitation*

To remove non-soluble proteins and fats, a carrez precipitation of the milk samples was done before the samples were analyzed via HPLC. Carrez I and Carrez II were prepared as known in the art. The sample (600 $\mu$L) was mixed with 75 $\mu$L carrez I, 75 $\mu$L carrez II, 150 $\mu$L NaOH (0.1 *M*) and 249 $\mu$L $H_2O_{dd}$. Then, the mixture was centrifuged (4 °C, 13000 rpm, 10 min) and the supernatant was filtered using Minisart SRP4 syringe filter (0.45 $\mu$m).

Analysis of the lactose hydrolysis in milk

Using BgaPW and Saphera 2600L, the lactose was under 1.0 g $L^{-1}$ (LOD) after 88 and 40 h, respectively. Whereas with Opti-B, still 7.9 g $L^{-1}$ lactose was detected after 144 h.

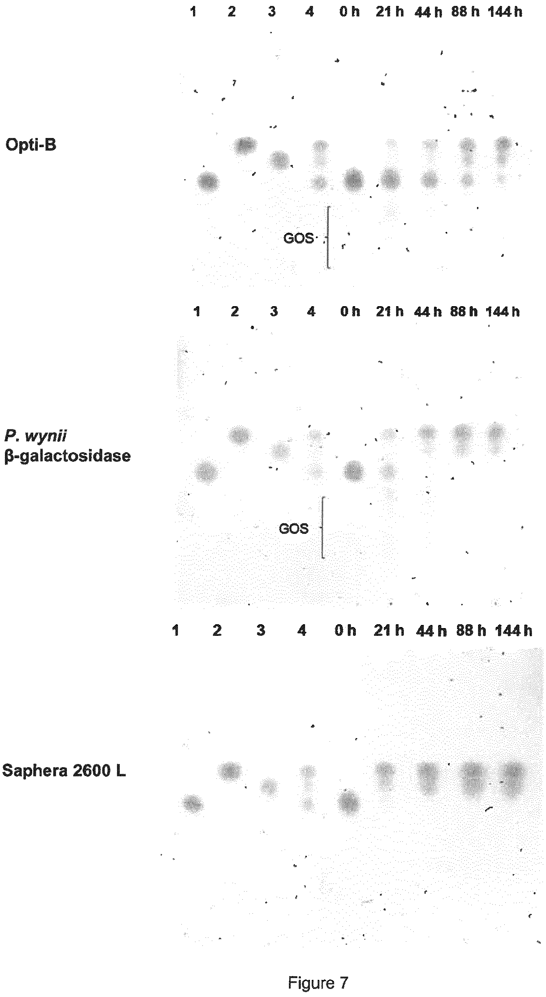

For qualitative analysis of the hydrolysis of lactose in milk and the GOS production during this process, a thin-layer chromatography was done. In Figure 7, the thin layer chromatography of the approaches with the three beta-galactosidases using 2.7 $nkat_{oNPGal,8°C}$ $mL^{-1}_{milk}$ are shown. On the lanes 1-4 the standard solutions of glucose and galactose and a mix of all sugars were applied.

The decrease of the lactose concentration was observed on the thin-layer chromatography of the hydrolysis with Opti-B. However, lactose was still detected after 144 h. The production of GOS as well as the further degradation of GOS was also observed. A very similar chromatogram was seen with *P. wynnii* beta-galactosidase, but lactose was hydrolyzed completely after 88 h. Furthermore, different GOS pattern were observed with the TLC of *P. wynnii* beta-galactosidase and Opti-B. With Opti-B, a main GOS spot was observed whereas with *P. wynnii* beta-galactosidase, different GOS spots were seen. Using Saphera 2600L for lactose hydrolysis resulted in a completely lactose conversion after 40 h without production of GOS. So, all thin-layer chromatographies confirm the HPLC analysis.

The difference of the kinetics between the three beta-galactosidases became very clear in the chromatographies. Both, Opti-B and *P. wynnii* beta-galactosidase, produce GOS during the process. However, different GOS were produced by Opti-B and BgaPW. Saphera 2600L, which is used in industry for production of lactose-free products, only hydrolyzes lactose.

**Claims**

1. Use of an enzyme having beta-galactosidase activity in the production of

   (i) lactose-depleted or lactose-free, and
   (ii) galacto-oligosaccharide-enriched

   dairy products, wherein said enzyme is derived from *Paenibacillus wynnii,* and wherein said enzyme comprises

   (a) the amino acid sequence of SEQ ID NO: 1; or
   (b) an amino acid sequence having at least 80% sequence identity to SEQ ID NO: 1 and having beta-galactosidase and transgalactosylation activity.

2. The use according to claim 1, wherein the enzyme consists of

   (a) the amino acid sequence of SEQ ID NO: 1; or
   (b) the amino acid sequence having at least 80% sequence identity to SEQ ID NO: 1 and having beta-galactosidase and transgalactosylation activity.

3. The use according to claim 1 or 2, wherein the amino acid sequence in (b) has at least 85%, at least 90%, at least 95%, at least 97%, at least 98%, at least 98.5%, at least 99%, at least 99.25%, at least 99.5%, at least 99.6%, at least 99.7%, at least 99.75%, at least 99.8%, at least 99.85%, at least 99.9%, or at least 99.95% sequence identity to SEQ ID NO: 1.

4. The use according to claim 1 or 2, wherein the enzyme consists of the amino acid sequence of SEQ ID NO: 1.

5. The use according to any one of claims 1 to 4, wherein the dairy product is milk.

**6.** A method for the production of a

   (i) lactose-depleted or lactose-free, and
   (ii) galacto-oligosaccharide-enriched

dairy product, comprising the step of incubating a dairy product with an enzyme having beta-galactosidase activity and being derived from *Paenibacillus wynnii* as defined in any one of present claims 1 to 4 under conditions and for a duration of time suitable to hydrolyse lactose present in said dairy product and to generate galacto-oligosaccharides in said dairy product.

**7.** The method according to claim 6, wherein the lactose content in the lactose-depleted or lactose-free dairy product, measured in accordance with the present description, is at most 0.1% (w/v).

**8.** The method according to claim 6 or 7, wherein the dairy product is milk.


**Patentansprüche**

**1.** Verwendung eines Enzyms, das Beta-Galactosidase-Aktivität aufweist, zur Herstellung von

   (i) laktosearmen oder laktosefreien, und
   (ii) Galacto-Oligosaccharid-angereicherten

Milchprodukten, wobei das Enzym von *Paenibacillus wynnii* stammt, und wobei das Enzym

   (a) die Aminosäuresequenz von SEQ ID NO: 1; oder
   (b) eine Aminosäuresequenz, die mindestens 80 % Sequenzidentität zu SEQ ID NO: 1 aufweist, und die Beta-Galactosidase- und Transgalactosylierungsaktivität aufweist,

umfasst.

**2.** Verwendung nach Anspruch 1, wobei das Enzym aus

   (a) der Aminosäuresequenz von SEQ ID NO: 1; oder
   (b) der Aminosäuresequenz, die mindestens 80 % Sequenzidentität zu SEQ ID NO: 1 aufweist, und die Beta-Galactosidase- und Transgalactosylierungsaktivität aufweist,

besteht.

**3.** Verwendung nach Anspruch 1 oder 2, wobei die Aminosäuresequenz in (b) mindestens 85%, mindestens 90%, mindestens 95%, mindestens 97%, mindestens 98%, mindestens 98,5%, mindestens 99%, mindestens 99,25%, mindestens 99,5%, mindestens 99,6%, mindestens 99,7%, mindestens 99,75%, mindestens 99,8%, mindestens 99,85%, mindestens 99,9% oder mindestens 99,95% Sequenzidentität zu SEQ ID NO: 1 aufweist.

**4.** Verwendung nach Anspruch 1 oder 2, wobei das Enzym aus der Aminosäuresequenz von SEQ ID NO: 1 besteht.

**5.** Verwendung nach einem der Ansprüche 1 bis 4, wobei das Molkereiprodukt Milch ist.

**6.** Verfahren zur Herstellung eines

   (i) laktosearmen oder laktosefreien, und
   (ii) Galacto-Oligosaccharid-angereicherten

Milchprodukts, umfassend den Schritt des Inkubierens eines Milchprodukts mit einem Enzym, das Beta-Galactosidase-Aktivität aufweist und von *Paenibacillus wynnii* stammt, wie in einem der vorliegenden Ansprüche 1 bis 4 definiert, unter Bedingungen und für eine Zeitdauer, die geeignet sind, in dem Milchprodukt vorhandene Lactose zu hydrolysieren und Galacto-Oligosaccharide in dem Milchprodukt zu erzeugen.

7. Verfahren nach Anspruch 6, wobei der Laktosegehalt in dem laktosearmen oder laktosefreien Milchprodukt, gemessen gemäß der vorliegenden Beschreibung, höchstens 0,1 % (w/v) beträgt.

8. Verfahren nach Anspruch 6 oder 7, wobei das Molkereiprodukt Milch ist.

**Revendications**

1. Utilisation d'une enzyme ayant une activité de bêta-galactosidase dans la production de

   (i) produits laitiers réduits en lactose ou exempts de lactose, et
   (ii) enrichis en galacto-oligosaccharides,

   dans laquelle ladite enzyme est dérivée de *Paenibacillus wynnii,* et dans laquelle ladite enzyme comprend

   (a) la séquence d'acides aminés de SEQ ID N° : 1 ; ou
   (b) une séquence d'acides aminés ayant au moins 80 % d'identité de séquence à SEQ ID N° : 1 et ayant une activité de bêta-galactosidase et transgalactosylation.

2. Utilisation selon la revendication 1, dans laquelle l'enzyme est constituée de

   (a) la séquence d'acides aminés de SEQ ID N° : 1 ; ou
   (b) la séquence d'acides aminés ayant au moins 80 % d'identité de séquence à SEQ ID N° : 1 et ayant une activité de bêta-galactosidase et transgalactosylation.

3. Utilisation selon la revendication 1 ou 2, dans laquelle la séquence d'acides aminés en (b) a au moins 85 %, au moins 90 %, au moins 95 %, au moins 97 %, au moins 98 %, au moins 98,5 %, au moins 99 %, au moins 99,25 %, au moins 99,5 %, au moins 99,6 %, au moins 99,7 %, au moins 99,75 %, au moins 99,8 %, au moins 99,85 %, au moins 99,9 % ou au moins 99,95 % d'identité de séquence à SEQ ID N° : 1.

4. Utilisation selon la revendication 1 ou 2, dans laquelle l'enzyme est constituée de la séquence d'acides aminés de SEQ ID N° : 1.

5. Utilisation selon l'une quelconque des revendications 1 à 4, dans laquelle le produit laitier est du lait.

6. Procédé pour la production d'un

   (i) produit laitier réduit en lactose ou exempt de lactose, et
   (ii) enrichi en galacto-oligosaccharides,

   comprenant l'étape d'incubation d'un produit laitier avec une enzyme ayant une activité de bêta-galactosidase et qui est dérivée de *Paenibacillus wynnii* telle que définie dans l'une quelconque des présentes revendications 1 à 4 dans des conditions et pendant une durée convenant à l'hydrolyse de lactose présent dans ledit produit laitier et pour générer des galacto-oligosaccharides dans ledit produit laitier.

7. Procédé selon la revendication 6, dans lequel la teneur en lactose dans le produit laitier réduit en lactose ou exempt de lactose, mesurée conformément à la présente description, est d'au plus 0,1 % (p/v).

8. Procédé selon la revendication 6 ou 7, dans lequel le produit laitier est du lait.

Figure 1

Figure 2

Figure 3

Figure 4

EP 3 911 180 B1

Figure 5

Figure 6

Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2530148 A1

- US 20170164631 A1

**Non-patent literature cited in the description**

- **SAMBROOK, J.; ; RUSSELL, D. W.** Molecular Cloning: A Laboratory Manual;. Cold Spring Harbour Laboratory Press, 2001, vol. 1